(19) Europäisches Patentamt / European Patent Office / Office européen des brevets

(11) **EP 4 349 887 A1**

(12) **EUROPEAN PATENT APPLICATION**
published in accordance with Art. 153(4) EPC

(43) Date of publication:
**10.04.2024 Bulletin 2024/15**

(21) Application number: **22820170.3**

(22) Date of filing: **06.06.2022**

(51) International Patent Classification (IPC):
$C08J\ 3/12^{(2006.01)}$    $C09K\ 11/06^{(2006.01)}$
$G01N\ 33/545^{(2006.01)}$    $G01N\ 21/64^{(2006.01)}$
$G01N\ 21/78^{(2006.01)}$

(52) Cooperative Patent Classification (CPC):
C08J 3/12; C09K 11/06; G01N 21/64; G01N 21/78;
G01N 33/545

(86) International application number:
**PCT/JP2022/022708**

(87) International publication number:
**WO 2022/259989 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.06.2021 JP 2021095971**

(71) Applicants:
 • **Canon Kabushiki Kaisha**
  **Tokyo 146-8501 (JP)**
 • **Canon Medical Systems Corporation**
  **Tochigi 324-0036 (JP)**

(72) Inventors:
 • **KAKEGAWA Norishige**
  **Tokyo 146-8501 (JP)**
 • **MASUMURA Takahiro**
  **Tokyo 146-8501 (JP)**
 • **SAKAKIBARA Teigo**
  **Tokyo 146-8501 (JP)**
 • **NAKAJIMA Ikuo**
  **Tokyo 146-8501 (JP)**
 • **YAMAUCHI Fumio**
  **Tokyo 146-8501 (JP)**
 • **KANAZAKI Kengo**
  **Tokyo 146-8501 (JP)**

(74) Representative: **WESER & Kollegen**
 **Patentanwälte PartmbB**
 **Radeckestraße 43**
 **81245 München (DE)**

(54) **POLARIZED LIGHT-EMITTING PARTICLES FOR SPECIMEN INSPECTION**

(57)    Provided is a particle having high detection sensitivity in the detection of a substance to be measured, such as an antigen or an antibody, from a body. The particle includes a rare earth complex, wherein the particle has a particle size distribution of 0.1 or less in terms of polydispersity index (pdi) measured by dynamic light scattering, and wherein the particle has a hydrophilic polymer on a surface thereof.

FIG. 1

EP 4 349 887 A1

**Description**

[Technical Field]

The present invention relates to a polarized luminescent particle for a specimen test and a method of producing the same.

[Background Art]

In the fields of medicine and clinical tests, high-sensitivity detection of a trace amount of a biological component from, for example, blood or a collected part of an organ is required for investigating the cause of a disease. Among detection techniques for biological components, immunoassays are widely utilized. As one of the immunoassays, there is known a latex agglutination method utilizing an antigen-antibody reaction. The "latex agglutination method" is such a method that, when an antigen serving as a target substance in a liquid such as a biological sample is detected, the antigen serving as the target substance is detected or quantified by mixing a latex particle having supported thereon, for example, an antibody that specifically binds to the antigen with the liquid, and measuring the degree of agglutination of the latex particle.

In the latex agglutination method, the antigen serving as the target substance is captured by the antibody serving as a target binding substance bound to the latex particle, and a plurality of the latex particles are crosslinked via the captured antigen, with the result that the agglutination occurs. That is, the amount of the antigen serving as the target substance in a liquid sample such as a biological sample can be quantified by evaluating the degree of the agglutination of the latex particle. The degree of the agglutination can be quantified by measuring and evaluating a change in amount of light transmitted through or scattered by the liquid sample.

The latex agglutination method can quantitatively evaluate the antigen as the target substance in a simple and rapid manner, but has involved a problem with detection limits in that the antigen cannot be detected when its amount in the liquid sample such as the biological sample is small.

In order to improve detection sensitivity for the target substance, for example, a system for scattering transmitted light for the liquid sample is replaced with a method for detection utilizing a luminescence characteristic with higher sensitivity.

Specifically, there has been proposed, for example, a specimen test method utilizing a fluorescence depolarization method (Patent Literature 1 and Patent Literature 2).

In Patent Literature 1, it is proposed that an apparatus for the fluorescence depolarization method be improved for a clinical purpose. The fluorescence depolarization method does not require a washing step called bound/free (B/F) separation including preliminary separation between a measurement substance and an unreacted luminescent substance, which imposes a problem in a fluorescence measurement method. Accordingly, a specimen test can be performed at the same level as that of the latex agglutination method. Further, it is conceived that the fluorescence depolarization method includes a measurement process including merely mixing a luminescent substance that specifically reacts with the measurement substance, and hence enables measurement to be performed by the same test system as that of the latex agglutination method. In Patent Literature 1, however, there is a proposal of use of a single molecule such as fluorescein as a luminescent material, which is applicable only to a drug, a low-molecular-weight antigen, and the like in principle.

In Patent Literature 2, with an aim to apply the fluorescence depolarization method to a macromolecule such as a protein in order to solve the problem of Patent Literature 1, i.e., the problem in that the fluorescence depolarization method can be applied only to a drug, a low-molecular-weight antigen, and the like, it is proposed to use, as a luminescent material, a material obtained by adsorbing a dye having a long-lifetime luminescence characteristic onto latex particles. In Patent Literature 2, it is proposed that even a macromolecule can be subjected to measurement by balancing a reduction in rotational Brownian motion of the substance in a liquid along with an increase in particle diameter and the length of luminescence lifetime based on the principle of the fluorescence depolarization method. However, in Patent Literature 2, bovine serum albumin (BSA), which is a biomolecule, is supported in order to suppress nonspecific adsorption onto surfaces of the particles, and hence a lot-to-lot variation may occur owing to a broadened particle size distribution and BSA, which is a protein. Accordingly, also in Patent Literature 2, it can hardly be said that detection of an antigen serving as the target substance with extremely high sensitivity has been achieved.

[Citation List]

[Patent Literature]

PTL 1: Japanese Patent Publication No. H03-52575
PTL 2: Japanese Patent No. 2893772

[Summary of Invention]

[Technical Problem]

The present invention has been made in view of such background art, and an object of the present invention is to provide a luminescent particle that enables a high-sensitivity specimen test by a fluorescence depolarization method, and a method of producing the same.

[Solution to Problem]

A particle according to the present invention is a particle including a rare earth complex,

wherein the particle has a particle size distribution of 0.1 or less in terms of polydispersity index (pdi value) measured by dynamic light scattering, and
wherein the particle has a hydrophilic polymer on a surface thereof.

Further, a particle according to the present invention is a particle including polystyrene and a siloxane bond,

wherein the particle has a hydrophilic polymer on a surface thereof,
wherein the particle contains a rare earth complex thereinside, and
wherein the particle has a particle size distribution of 0.1 or less in terms of polydispersity index (pdi value) measured by dynamic light scattering.

[Advantageous Effects of Invention]

According to the particle of the present invention, a reaction in which the particle aggregates in a liquid can be detected with high sensitivity by polarized light measurement.
In addition, with a hydrophilic layer formed on the surface of the particle, the particle is conceived to have a high suppressing effect on nonspecific adsorption without using BSA. Accordingly, the particle of the present invention provides high sensitivity when used in the fluorescence depolarization method.

[Brief Description of Drawings]

[FIG. 1]

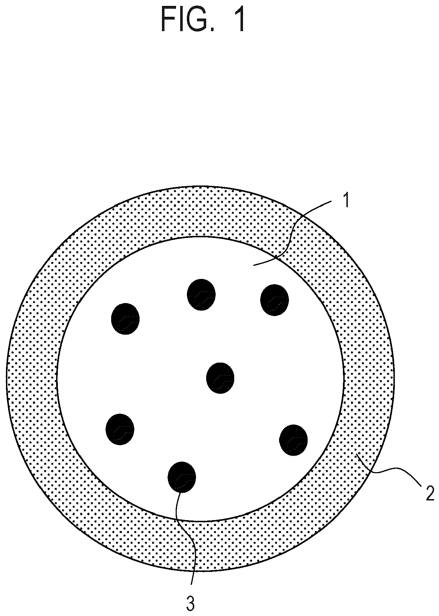

FIG. 1 is a schematic view for illustrating the structure of a particle according to an embodiment of the present invention.
[FIG. 2]

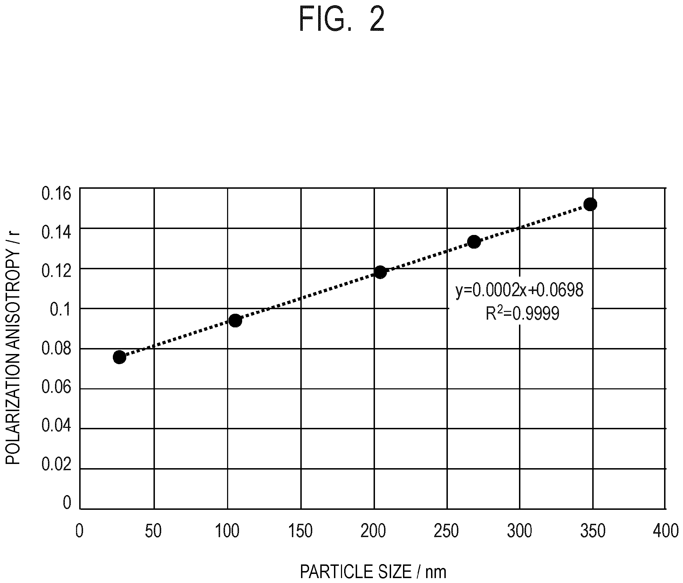

FIG. 2 is an explanatory graph of the relationship of values of polarization anisotropy "r" determined from the results of fluorescence polarization measurement of samples of Examples 1, 2, 3, 4, and 5 with their particle sizes.
[FIG. 3]

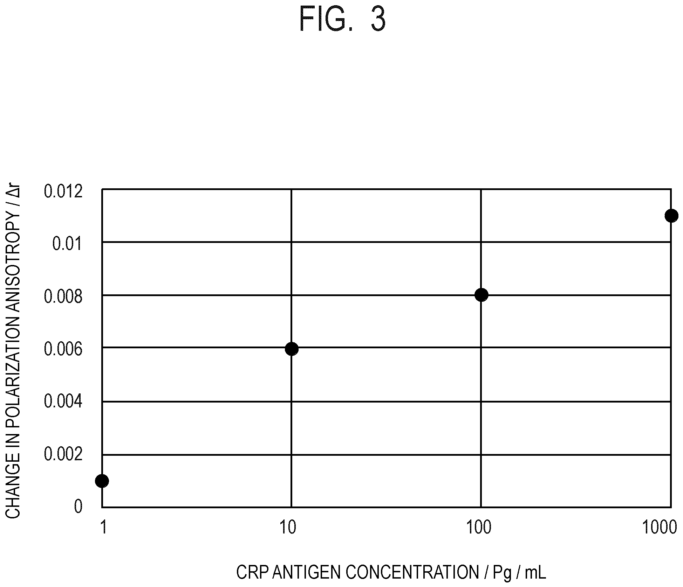

FIG. 3 is an explanatory graph of the results of quantification of an antigen-antibody reaction using a CRP antibody, which was evaluated in Example 6, through use of a fluorescence depolarization method.

[Description of Embodiments]

Preferred embodiments of the present invention are described in detail below. However, the embodiments are not intended to limit the scope of the present invention.
An example of a particle according to this embodiment is described in detail with reference to FIG. 1.
As illustrated in FIG. 1, the particle of the present invention includes a particle substrate 1 containing a rare earth complex 3, and a hydrophilic layer 2 coating the surface thereof.
The particle according to this embodiment has a small particle size distribution, and the surface of the particle is hydrophilically coated.
A rare earth complex showing polarization anisotropy is present inside the particle.

FIG. 1 is a schematic view for illustrating an example of the particle according to this embodiment. The particle in FIG. 1 (which may also be referred to as "luminescent particle") is spherical. The luminescent particle has an average particle diameter of 25 nm or more and 400 nm or less, and a polydispersity index (hereinafter abbreviated as "pdi") of 0.1 or less as measured by a dynamic light scattering method.

When particles dispersed in a solution are irradiated with laser light and the resultant scattered light is observed with a photon detector, the particles are constantly shifting their positions by Brownian motion, and hence an intensity distribution based on interference of the scattered light also constantly fluctuates. The dynamic light scattering method is a measurement method including observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived. In addition, when the autocorrelation function is subjected to cumulant analysis, a cumulant diameter and the pdi can be determined. The pdi indicates the polydispersity of a particle diameter distribution, and a smaller numerical value thereof indicates that the particle size distribution is more uniform.

It is considered that, in general, particles having a pdi of 0.1 or less are monodispersed in a solvent.

The particle according to this embodiment needs to have a pdi of 0.1 or less, which is suitably as small as possible. In order for the particle to be able to stably have a pdi of less than 0.1, it is desired that nothing be provided on the surface of the particle. However, nonspecific adsorption of substances other than the target onto the particle needs to be prevented, and hence a coating for keeping the surface of the particle hydrophilic is needed.

Meanwhile, it is difficult to stably keep the pdi at 0.1 or less by a generally used method including supporting BSA. The method including supporting BSA may also cause a lot-to-lot variation. In view of this, such a coating on the surface of the particle that the pdi for the particle size becomes 0.1 or less through use of a hydrophilic polymer is needed.

The luminescent particle according to this embodiment has an average particle diameter of 25 nm or more and 400 nm or less, suitably 50 nm or more and 400 nm or less. A particle having an average particle diameter of 50 nm or more and 200 nm or less is more suitably used. When the average particle diameter is 400 nm or more, polarization anisotropy before aggregation is increased to reduce its difference from polarization anisotropy after an aggregation reaction. In addition, when the average particle diameter is 25 nm or less, the change in size of the particle diameter at the time of an antigen-antibody reaction is reduced, and hence it becomes difficult to grasp a change in polarization anisotropy by fluorescence depolarization of phosphorescence. The term "average particle diameter" as used herein refers to a number-average particle diameter, and the average particle diameter may be measured by a dynamic light scattering method.

By reducing the particle size distribution of particles and introducing a rare earth complex showing polarized luminescence into the particle, a change in polarized luminescence characteristic can be grasped even when a slight change occurs in the dispersion state of the particles in a liquid. Specifically, even if the concentration of an antigen in a solution is from about a nanogram to about a picogram per mL, when a sandwich-type antigen-antibody reaction in which the particles aggregate via the antigen occurs, a change in rotational Brownian motion of the particles can be grasped as a change in polarization anisotropy.

The rare earth complex showing polarization anisotropy can be used to emit polarized light that is phosphorescence having a long lifetime. The term "polarization anisotropy" as used herein means the presence of anisotropy in transition moment (transition dipole moment). In general, the polarized luminescence means that, in the case of a luminescent dye having anisotropy in transition moment, when polarized light along its transition moment is used as excitation light, its luminescence is also polarized light along the transition moment. In the case of the rare earth complex, when a ligand having anisotropy is excited, fluorescent luminescence based on energy transfer from the ligand to the central metal ion is shown, and hence polarized light is emitted when the ligand is excited with polarized light.

The principle of the fluorescence depolarization is the measurement of a shift in transition moment based on the rotational motion of a luminescent material during the occurrence of the polarized luminescence. The rotational motion of the luminescent material may be expressed by the equation (1):

$$Q = 3V\eta/kT \cdots (1)$$

where Q represents the rotational relaxation time of the material, V represents the volume of the material, $\eta$ represents the viscosity of a solvent, k represents the Boltzmann constant, and T represents an absolute temperature.

The rotational relaxation time of the material is a period of time required for a molecule to rotate by an angle $\theta$ (68.5°) at which $\cos\theta = 1/e$.

It is found from the equation (1) that the rotational relaxation time of the luminescent material is proportional to the volume of the material, that is, the cube of the particle diameter. Meanwhile, a relationship between the luminescence lifetime of the material and the degree of polarization in the fluorescence depolarization may be expressed by the equation

(2):

$$p0/p = 1 + A(\tau/Q) \cdots (2)$$

where p0 represents a degree of polarization at a time when the material is stationary (Q=∞), "p" represents the degree of polarization, A is a constant, $\tau$ represents the luminescence lifetime of the material, and Q represents the rotational relaxation time.

According to the equation (1) and the equation (2), in order for a change in degree of polarization to be measured large, a relationship between the luminescence lifetime of the luminescent material and its rotational relaxation time, that is, the volume (particle diameter) of the luminescent material, is important, and as the particle diameter of the luminescent material increases, the luminescence lifetime needs to be lengthened as well.

When the degree of polarization of the luminescence represented by the equation (2) is determined experimentally, it is appropriate that polarized light be allowed to enter a sample, and luminescence be detected in a 90° direction with respect to the traveling direction and vibration direction of excitation light. In this case, it is appropriate that the detected light be detected by being divided into polarized light components in parallel and perpendicular directions with respect to the polarized light that is the incident light, and the degree of polarization be evaluated with the equation represented by the equation (3):

$$r(t) = (I\|(t) - GI\perp(t))/(I\|(t) + 2GI\perp(t)) \cdots (3)$$

where r(t) represents a polarization anisotropy at a time "t", I∥(t) represents the luminescence intensity of a luminescence component parallel to the excitation light at the time "t", I⊥(t) represents the luminescence intensity of a luminescence component perpendicular to the excitation light at the time "t", and G represents a correction value, the ratio of I⊥/I∥ measured with excitation light having a vibration direction different by 90° from that of the excitation light used for sample measurement.

That is, when the particle size and the luminescence lifetime fall within appropriate ranges, a change in particle size of the luminescent material through, for example, an antigen-antibody reaction can be sensitively read as a value for polarization anisotropy. The polarization anisotropy refers to a value for the degree of polarization corrected with G and 2G, and the degree of polarization is a value obtained by removing G and 2G from the equation (3). In actual measurement, the correction value G is required, and hence the polarization anisotropy is determined.

Further, it is preferred that the luminescent particle of the present invention have a polarization anisotropy <r> of 0.1 or more, which is determined by the following equation (4).

[Math. 1]

$$\langle r \rangle = \frac{I_{vv} - G \cdot I_{VH}}{I_{vv} + 2 \cdot G \cdot I_{VH}}$$

$$G = \frac{I_{HV}}{I_{HH}}$$

$$\cdots \text{(Equation 4)}$$

in the equation (4),

<r> represents the polarization anisotropy,

Ivv represents the luminescence intensity of a luminescence component having a vibration direction parallel to that of a first polarized light beam at the time of excitation by the first polarized light beam,

$I_{VH}$ represents the luminescence intensity of a luminescence component having a vibration direction orthogonal to that of the first polarized light beam at the time of excitation by the first polarized light beam,

$I_{HV}$ represents the luminescence intensity of a luminescence component having a vibration direction orthogonal to that of a second polarized light beam having a vibration direction orthogonal to that of the first polarized light beam at the time of excitation by the second polarized light beam,

$I_{HH}$ represents the luminescence intensity of a luminescence component having a vibration direction parallel to that of the second polarized light beam having a vibration direction orthogonal to that of the first polarized light beam at the time of excitation by the second polarized light beam, and
G represents a correction value.

(Particle Substrate 1)

The particle substrate 1 in FIG. 1 is spherical. A material therefor is not particularly specified as long as the rare earth complex showing polarization anisotropy can be stably incorporated thereinto, but in particular, a polystyrene particle obtained by polymerizing a styrene monomer as a main component, a particle obtained by incorporating a polymer containing a siloxane bond into the polystyrene particle, or the like may be suitably used. The polystyrene particles may be produced as particles having an extremely uniform particle size distribution by an emulsion polymerization method to be described later, and the hydrophilic layer to be described later or a ligand may be provided via a silanol present in the polymer containing a siloxane bond.

(Hydrophilic Layer 2)

A hydrophilic layer 2 includes a molecule or polymer containing a hydrophilic group. The "molecule containing a hydrophilic group" means a molecule or polymer having a hydroxy group, an ether, pyrrolidone, a betaine structure, or the like. Specifically, the hydrophilic layer 2 contains, as a main component, for example, polyethylene glycol, polyvinylpyrrolidone, a polymer of sulfobetaine or phosphobetaine, or polyglycidyl methacrylate whose molecule has an end modified with a hydroxy group by ring-opening a glycidyl group. Alternatively, a single molecule having a hydrophilic group may be provided on the surface of a silica particle through use of a silane coupling agent or the like. The thickness of the hydrophilic layer 2 is not limited, but does not need to be set to be large beyond a thickness with which hydrophilicity can be exhibited. When the hydrophilic layer is excessively thick, the hydrophilic layer may become hydrogel-like, and the thickness of the hydrophilic layer hydrated by the influence of ions in the solvent may change. The thickness of the hydrophilic layer is suitably 1 nm or more and 50 nm or less.

(Rare Earth Complex 3)

A rare earth complex showing polarization anisotropy is used as a luminescent dye in the present invention because of the following feature: the wavelength and intensity of its luminescence are hardly influenced by the surroundings, and hence the luminescence has a long lifetime. A rare earth complex 3 includes a rare earth element and a ligand. The rare earth element is selected from europium, terbium, neodymium, erbium, yttrium, lanthanum, cerium, samarium, gadolinium, dysprosium, thulium, ytterbium, scandium, and the like. In consideration of, for example, the luminescence lifetime and a visible luminescence wavelength region, europium or terbium may be suitably used.

For example, europium generally has a luminescence lifetime of from 0.1 ms to 1.0 ms. The luminescence lifetime and the rotational relaxation time obtained from the equation (1) need to be appropriately adjusted. In the case of europium in a water dispersion, when its particle size is an average particle diameter of from about 50 nm to about 300 nm, the polarization anisotropy represented by the equation (3) significantly changes before and after the antigen-antibody reaction.

At least one of the constituent ligands of the rare earth complex 3 is a ligand having a light-collecting function. The "light-collecting function" refers to an action of being excited at a particular wavelength to excite the central metal of the complex through energy transfer. Further, a molecule having anisotropy in transition moment is selected as the ligand having a light-collecting function. For example, phenanthroline is suitably used. However, the ligand having a light-collecting function is not limited thereto. In addition, it is preferred that the constituent ligands of the rare earth complex 3 include a ligand such as a β-diketone to prevent coordination of a water molecule. The ligand such as the β-diketone coordinated to a rare earth ion suppresses a deactivation process due to the transfer of energy to a solvent molecule or the like to provide strong fluorescence luminescence.

The rare earth complex 3 may be a polynuclear complex as long as the complex shows polarization anisotropy.

The polarization anisotropy of the rare earth complex 3 is represented by the equation (3). At the time of a state in which the Brownian rotational motion of the rare earth complex 3 can be regarded as stationary in a medium, the polarization anisotropy is desirably 0.1 or more. The state in which the Brownian rotational motion can be regarded as stationary refers to a state in which the rotational relaxation time of the particles is sufficiently longer than the luminescence lifetime of the rare earth complex 3.

When a liquid having dispersed therein the particle according to this embodiment is used, the anisotropy of polarized luminescence can be detected with high sensitivity with respect to the aggregation/dispersion behavior of the particle. Accordingly, a colloidal liquid obtained by dispersing the particle according to this embodiment in an aqueous solvent can be utilized as a high-sensitivity antibody test reagent through use of a fluorescence depolarization method. A buffer solution may be used as the aqueous solvent. In addition, a surfactant, a preservative, a sensitizer, or the like may be added into the aqueous solvent in order to enhance the stability of the liquid having dispersed therein the particle according to this embodiment.

(Method of producing Particle)

Next, an example of a method of producing the particle according to this embodiment is described.

The method of producing the particle according to this embodiment includes a step (first step) of mixing a radically polymerizable monomer, a radical polymerization initiator, a polarized luminescent rare earth complex, and a hydrophilic polymer with an aqueous medium to prepare an emulsion.

Further, the method includes a step (second step) of heating the emulsion to polymerize the monomer.

The luminescent particle according to this embodiment is a luminescent particle obtained by copolymerizing a compound having a double bond having radical polymerizability.

Further, the method includes a step (third step) of providing a functional group capable of bonding a ligand to be described later on the surface of the luminescent particle. In this step, any one of a carboxy group, an amino group, a thiol group, an epoxy group, a maleimide group, a succinimidyl group, or a silicon alkoxide group may be used as the functional group capable of bonding a ligand.

(1. Radically Polymerizable Monomer)

The radically polymerizable monomer is characterized by including at least a styrene-based monomer.

The radically polymerizable monomer may further include a monomer selected from the group consisting of an acrylate-based monomer; and a methacrylate-based monomer. Examples thereof may include butadiene, vinyl acetate, vinyl chloride, acrylonitrile, methyl methacrylate, methacrylonitrile, and methyl acrylate. In addition to the styrene-based monomer, at least one selected from the group of the above-mentioned monomers may be used. That is, those monomers may be used alone or in combination thereof. In addition, a monomer having two or more double bonds per molecule such as divinylbenzene may be used as a crosslinking agent.

In addition, a radically polymerizable monomer containing an organic silane may be used in order to incorporate a siloxane bond into the luminescent particle. An organic silane compound may be, for example, vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, or 3-acryloxypropyltrimethoxysilane. At least one selected from the group of those monomers may be used. That is, those organic silane compounds may be used alone or in combination thereof. The radically polymerizable monomer containing an organic silane plays a role in forming a backbone of an inorganic oxide in the synthesized luminescent particle to improve the physical and chemical stability of the luminescent particle. Further, the radically polymerizable monomer containing an organic silane plays a role in enhancing affinity between a unit having a functional group capable of bonding the hydrophilic polymer on the surface of the luminescent particle to a ligand and a backbone material for a polymer fine particle including styrene.

Further, the use of the radically polymerizable monomer containing an organic silane can provide a silanol group on the surface of the luminescent particle. With a hydrogen bond between the silanol group and the polymer showing hydrophilicity such as PV polyvinylpyrrolidone, the polyvinylpyrrolidone is more strongly adsorbed onto the luminescent particle surface.

(Radical Polymerization Initiator)

A wide range of compounds selected from, for example, azo compounds and organic peroxides may each be used as the radical polymerization initiator. Specific examples thereof may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

(Hydrophilic Polymer)

The hydrophilic polymer for suppressing nonspecific adsorption is a hydrophilic polymer containing a unit having an ether, a betaine, a pyrrolidone ring, or the like, is contained in the synthesized luminescent particle, and is mainly present on the particle surface. The polymer having a pyrrolidone ring is hereinafter abbreviated as "PVP". When the PVP is fed at the time of the synthesis of the polymer fine particle, a nonspecific adsorption-suppressing ability and a ligand-bonding ability can be simultaneously imparted to the polymer fine particle. That is, this can be achieved by mixing a radically polymerizable monomer including styrene, a radical polymerization initiator, and a copolymer containing a unit having a pyrrolidone ring and a unit having a functional group capable of bonding a ligand with an aqueous medium to prepare an emulsion.

The PVP fed at the time of the synthesis is more hydrophilic than the radically polymerizable monomer, and hence is present at an interface between the solvent and the polymer fine particle that is being produced at the time of the synthesis. The polymer fine particle adsorbs the PVP onto the surface of the particle by involving part of the polymer chain of the PVP at the time of the synthesis, or by physical/chemical adsorption such as an interaction between a pyrrolidone ring and styrene (radically polymerizable monomer). In addition, the luminescent particle may contain a siloxane bond, and the ratio of a unit having a siloxane bond to the styrene monomer is preferably 40 wt% or less.

The molecular weight of the PVP is preferably 10,000 or more and 100,000 or less, more suitably 40,000 or more and 70,000 or less. When the molecular weight is less than 10,000, the hydrophilicity of the surface of the luminescent particle is weak, and hence nonspecific adsorption is liable to occur. When the molecular weight is more than 100,000, the hydrophilic layer on the surface of the luminescent particle becomes excessively thick, with the result that the luminescent particle gelates and becomes difficult to handle.

In addition to the PVP, another hydrophilic polymer may be added as a protective colloid at the time of the synthesis of the luminescent particle.

In addition, the particle has a difference between absorption spectra of 0.1 or less at an optical path of 10 mm and a wavelength of 572 nm before and after 30 $\mu$L of a dispersion of 0.1 wt% of the particle is added to 60 $\mu$L of a buffer solution mixed with 16 $\mu$L of serum and the mixture is left to stand at 37°C for 5 minutes. A particle showing such difference between the absorption spectra has little nonspecific adsorption of impurities in serum, and hence is preferred.

(Aqueous Medium)

The aqueous solution (aqueous medium) preferably contains water at 80% or more and 100% or less in the medium. Examples of the aqueous solution include water and a solution obtained by mixing water with a water-soluble organic solvent, such as methanol, ethanol, isopropyl alcohol, or acetone. When an organic solvent other than water is incorporated at more than 20%, dissolution of the polymerizable monomer may occur at the time of the production of the luminescent particle.

In addition, when the radically polymerizable monomer containing an organic silane is used, the aqueous solution (aqueous medium) preferably has its pH adjusted to 6 or more and 9 or less in advance. When the pH has a value of less than 6 or more than 9, an alkoxide group or a silanol group in the organic silane compound may undergo condensation polymerization or a reaction with another functional group before the formation of the luminescent particle, leading to aggregation of the particles to be obtained. In this embodiment, the alkoxide is not intentionally subjected, before the formation of the polymer fine particle, to condensation polymerization.

The pH is preferably adjusted with a pH buffer, but may be adjusted with an acid or a base.

Other than the foregoing, a surfactant, an antifoaming agent, a salt, a thickener, and the like may be used by being added at a ratio of 10% or less with respect to the aqueous medium.

In a method of producing the polymer fine particle according to this embodiment, first, the PVP be dissolved in the aqueous medium adjusted to a pH of from 6 to 9. The content of the PVP is from 0.01 wt% to 10 wt%, preferably from 0.03 wt% to 5 wt% with respect to the aqueous medium. When the content is 0.01 wt% or less, the amount of adsorption onto the polymer fine particle is small, and the effect thereof is not expressed. In addition, when the content is 10 wt% or more, the viscosity of the aqueous medium may be increased to preclude sufficient stirring.

Subsequently, a radically polymerizable monomer (A) is added into the above-mentioned aqueous medium to prepare an emulsion. At this time, the emulsion may be prepared by also adding a radically polymerizable monomer (B) containing an organic silane into the aqueous medium in addition to the radically polymerizable monomer (A). A weight ratio between the radically polymerizable monomer (A) and the radically polymerizable monomer (B) containing an organic silane is from 6:4 to 10:0.

Further, the prepared monomer liquid is mixed with the polarized luminescent rare earth complex. At this time, when the solubility of the polarized luminescent rare earth complex is low, a water-insoluble organic solvent may be added. A weight ratio between the polarized luminescent rare earth complex and the monomers is from 1:1,000 to 1:10.

When the ratio of the radically polymerizable monomer (A) is 6 or less, the specific gravity of the particle as a whole may be increased, resulting in remarkable sedimentation of the particle. In addition, the radically polymerizable monomer (B) containing an organic silane is not necessarily required to be added, but in order to increase adhesiveness between the PVP and the luminescent particle, it is more suitable that the radically polymerizable monomer (B) containing an organic silane be added.

A weight ratio between the aqueous medium and the total amount of the radically polymerizable monomer (A) and the radically polymerizable monomer (B) containing an organic silane is preferably from 5:5 to 9.5:0.5. When the ratio of the aqueous medium is 5 or less, remarkable aggregation of the luminescent particle to be produced may occur. In addition, even when the ratio of the aqueous medium is 9.5 or more, there is no problem with the production of the luminescent particle, but the production amount thereof may be reduced.

The radical polymerization initiator is used by being dissolved in water, a buffer, or the like. The radical polymerization initiator may be used between 0.5 mass% and 10 mass% with respect to the total weight of the radically polymerizable monomer (A) and the radically polymerizable monomer (B) containing an organic silane.

In the above-mentioned step of heating the emulsion, it is only required that the entire emulsion be uniformly heated. A heating temperature may be arbitrarily set between 50°C and 80°C, and a heating time may be arbitrarily set between 2 hours and 24 hours. Through the heating of the emulsion, the monomers are polymerized.

The functional group capable of bonding a ligand is not particularly limited as long as the functional group can bond an antibody, an antigen, an enzyme, or the like, but examples thereof include a carboxy group, an amino group, a thiol group, an epoxy group, a maleimide group, a succinimidyl group, and a silicon alkoxide group. For example, a silane coupling agent having the functional group capable of bonding a ligand and the synthesized luminescent particle may be mixed to provide the functional group on the luminescent particle surface. Specifically, an aqueous solution of a silane coupling agent having a carboxy group may be prepared and mixed with a dispersion of the synthesized luminescent particle to provide the carboxy group on the luminescent particle surface. At this time, a dispersant such as Tween 20 may be added to the reaction solution. A reaction temperature may be arbitrarily set between 0°C and 80°C, and a reaction time may be arbitrarily set between 1 hour and 24 hours. In order to suppress an abrupt condensation reaction of the silane coupling agent, it is suitable that the temperature be set to be equal to or lower than a room temperature of about 25°C, and the reaction time be set to from about 3 hours to about 14 hours. Depending on the functional group capable of bonding a ligand, the reaction with the luminescent particle surface may be promoted by adding an acid or alkali catalyst.

The particle according to this embodiment can be utilized as a particle for a specimen test by bonding a ligand such as any of various antibodies thereto. An optimal technique for bonding an antibody of interest through utilization of a functional group present on the hydrophilic layer 2 only needs to be selected.

(Ligand/Affinity Particle)

In this embodiment, there can be provided an affinity particle including the particle according to this embodiment and a ligand bonded to a reactive functional group.

In this embodiment, the "ligand" refers to a compound that specifically binds to the receptor of a particular target substance. The ligand binds to the target substance at a predetermined site and has selectively or specifically high affinity. Examples of a combination of the target substance and the ligand, or the ligand and the target substance include: an antigen and an antibody; an enzyme protein and a substrate thereof; a signal substance, such as a hormone or a neurotransmitter, and a receptor thereof; and nucleic acids. However, the ligand in this embodiment is not limited thereto. Examples of the nucleic acids include deoxyribonucleic acids. The affinity particle in this embodiment has selectively or specifically high affinity for the target substance. The ligand in this embodiment is preferably any one of an antibody, an antigen, and a nucleic acid.

In this embodiment, a hitherto known method may be applied to a chemical reaction for chemically bonding the reactive functional group of the particle according to this embodiment and the ligand to the extent that the object of the present invention can be achieved. In addition, when the ligand is amide-bonded, a catalyst such as 1-[3-(dimethylaminopropyl)-3-ethylcarbodiimide] may be appropriately used.

When using an antibody (antigen) as the ligand and an antigen (antibody) as the target substance, the affinity particle in this embodiment may be preferably applied to a latex immunoagglutination measurement method utilized widely in the fields of clinical tests, biochemical research, and the like.

(Test Reagent for In Vitro Diagnosis)

A test reagent for in vitro diagnosis, that is, a test reagent for use in the detection of a target substance in a specimen by in vitro diagnosis in this embodiment includes the affinity particle according to this embodiment and a dispersion medium for dispersing the affinity particle. The amount of the affinity particle according to this embodiment to be incorporated into the test reagent in this embodiment is preferably from 0.000001 mass% to 20 mass%, more preferably from 0.0001 mass% to 1 mass%. The test reagent according to this embodiment may include, in addition to the affinity particle according to this embodiment, a third substance, such as a solvent or a blocking agent, to the extent that the object of the present invention can be achieved. The reagent may include a combination of two or more kinds of third substances, such as a solvent and a blocking agent. Examples of the solvent to be used in this embodiment include various buffer solutions, such as a phosphate buffer solution, a glycine buffer solution, a Good's buffer solution, a Tris buffer solution, and an ammonia buffer solution, but the solvent in the test reagent in this embodiment is not limited thereto.

When the test reagent in this embodiment is used for the detection of an antigen or an antibody in a specimen, an antibody or an antigen may be used as the ligand.

(Test Kit)

A test kit for use in the detection of a target substance in a specimen by in vitro diagnosis in this embodiment includes the above-mentioned reagent and a case enclosing the reagent. The kit according to this embodiment may contain a sensitizer for promoting the aggregation of the particle at the time of an antigen-antibody reaction. Examples of the sensitizer include polyvinyl alcohol, polyvinylpyrrolidone, and polyalginic acid, but the present invention is not limited thereto. In addition, the test kit according to this embodiment may include a positive control, a negative control, a serum diluent, or the like. As a medium for the positive control or the negative control, there may be used serum free of a measurable target substance, physiological saline, or a solvent. The test kit according to this embodiment may be used for the method of detecting a target substance according to this embodiment in the same manner as a kit for use in general detection of a target substance in a specimen by in vitro diagnosis. In addition, the concentration of the target substance may also be measured by a hitherto known method, and in particular, the test kit is suitably used for the detection of a target substance in a specimen by a latex agglutination method.

(Detection Method)

A method of detecting a target substance in a specimen by in vitro diagnosis in this embodiment includes a step of mixing the affinity particle according to this embodiment with a specimen that may contain the target substance. In addition, the mixing of the affinity particle according to this embodiment with the specimen is preferably performed within the range of from pH 3.0 to pH 11.0. In addition, a mixing temperature falls within the range of from 20°C to 50°C, and a mixing time falls within the range of from 1 minute to 60 minutes. In addition, this detection method preferably uses a solvent. In addition, the concentration of the affinity particle according to this embodiment in the detection method according to this embodiment is preferably from 0.000001 mass% to 1 mass%, more preferably from 0.00001 mass% to 0.001 mass% in a reaction system. In the method of detecting a target substance in a specimen according to this embodiment, an aggregation reaction occurring as a result of the mixing of the affinity particle according to this embodiment with the specimen is preferably detected by a fluorescence depolarization method. Specifically, the method includes the steps of: mixing a test reagent with a specimen to provide a mixed liquid; irradiating the mixed liquid with polarized light; and separately detecting polarized light components of luminescence of the affinity particle in the mixed liquid.
Through optical detection of the above-mentioned aggregation reaction occurring in the mixed liquid, the target substance in the specimen is detected, and further, the concentration of the target substance may also be measured.

[Examples]

The present invention is specifically described below by way of Examples. However, the present invention is not limited to these Examples.

(1) Production of Luminescent Particles 1 to 8

Polyvinylpyrrolidone (PVP-K30: manufactured by Tokyo Chemical Industry Co., Ltd.) and sodium dodecyl sulfate (manufactured by Wako Pure Chemical Industries, Ltd., hereinafter abbreviated as "SDS") were dissolved in a 2-morpholinoethanesulfonic acid (MES) buffer solution (manufactured by Kishida Chemical Co., Ltd.) having a pH of 7 to prepare a solvent A. A polarized luminescent rare earth complex [tris(2-thenoyltrifluoroacetonato)(1,10-phenanthroline)europium(III)] (manufactured by Central Techno Corporation hereinafter abbreviated as "Eu(TTA)$_3$Phen"), a styrene monomer (manufactured by Kishida Chemical Co., Ltd.) and 3-methacryloxypropyltrimethoxysilane (manufactured by Tokyo Chemical Industry Co., Ltd., hereinafter abbreviated as "MPS") were mixed to prepare a reaction liquid B. The reaction liquid B was added into a four-necked flask containing the solvent A, and the mixture was stirred with a mechanical stirrer set to 300 rpm. After 30 minutes of stirring under a nitrogen flow condition, the temperature of an oil bath that had been prepared was set to 70°C, and the nitrogen flow was performed for an additional 30 minutes. After the heating and stirring of the mixed liquid, an aqueous solution having dissolved therein potassium persulfate (hereinafter abbreviated as "KPS") (manufactured by Sigma-Aldrich) was added into the reaction solution, and emulsion polymerization was performed for 10 hours. After the polymerization reaction, the resultant suspension was centrifuged, the supernatant was removed, and the precipitate was redispersed with pure water. The operations of centrifugation and redispersion were repeated 3 times to wash the product. The resultant precipitate was redispersed with pure water to provide a dispersion of each of luminescent particles 1 to 8. The mass ratios of the respective reagents used in the production of the luminescent particles 1 to 8 are shown in Table 1.
In the production of the luminescent particles 5, SDS was added together with PVP-K30. Further, in the production of the luminescent particles 7 and the luminescent particles 8, sodium p-styrenesulfonate (manufactured by Tokyo Chemical Industry Co., Ltd., hereinafter abbreviated as "PSSNa") was added at a mass ratio of 0.008.

[Table 1]

Table 1

| Sample | Solvent A | | Reaction liquid B | | | Catalyst |
|---|---|---|---|---|---|---|
| | Solvent amount | PVP-K30 | Eu (TTA)$_3$Phen | Styrene monomer | MPS | KPS |
| Luminescent particles 1 | 160 | 1.3 | 0.05 | 3.2 | 1 | 0.4 |
| Luminescent particles 2 | 80 | 0.7 | 0.015 | 3.2 | 1 | 0.01 |
| Luminescent particles 3 | 80 | 0.7 | 0.015 | 4.2 | 1 | 0.01 |
| Luminescent particles 4 | 80 | 0.7 | 0.015 | 4.2 | 1 | 0.005 |
| Luminescent particles 5 | 80 | 0.7 plus SDS at 0.5 | 0.015 | 4.2 | 1 | 0.2 |
| Luminescent particles 6 | 80 | 0.7 | 0.015 | 4 | 1.2 | 0.05 |
| Luminescent particles 7 | 80 | PSSNa at 0.008 in place of PVP-K30 | 0.015 | 4 | 1.2 | 0.05 |
| Luminescent particles 8 | 80 | | 0.015 | 4.2 | 1 | 0.01 |

An aliquot of the dispersion of each of the luminescent particles 1 to 8 obtained by the emulsion polymerization was taken and added to an aqueous solution having dissolved therein 1 mass% of Tween 20 (manufactured by Kishida Chemical Co., Ltd.). After 10 minutes of stirring, a silane coupling agent X12-1135 (manufactured by Shin-Etsu Chemical Co., Ltd.) was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water. Thus, the luminescent particles 1 to 8 were obtained. A mass ratio among the luminescent particles, pure water, and X12-1135 loaded was set to 1:300:2.

(Example 1)

A particle dispersion in which a sample corresponding to the synthesized luminescent particles 5 was diluted with pure water to a concentration of 0.001 mg/mL was prepared.

(Example 2)

A particle dispersion in which a sample corresponding to the synthesized luminescent particles 1 was diluted with pure water to a concentration of 0.001 mg/mL was prepared.

(Example 3)

A particle dispersion in which a sample corresponding to the synthesized luminescent particles 2 was diluted with pure water to a concentration of 0.001 mg/mL was prepared.

(Example 4)

A particle dispersion in which a sample corresponding to the synthesized luminescent particles 3 was diluted with pure water to a concentration of 0.001 mg/mL was prepared.

(Example 5)

A particle dispersion in which a sample corresponding to the synthesized luminescent particles 4 was diluted with pure water to a concentration of 0.001 mg/mL was prepared.

(Example 6)

(Production of Anti-CRP Antibody-modified Affinity Particles)

An aliquot of 0.25 mL of the luminescent particle dispersion at 1.2 wt% corresponding to the synthesized luminescent particles 6 was taken, and the solvent was replaced by 1.6 mL of a MES buffer solution having a pH of 6.0. To the luminescent particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-CRP antibody was added at 100 µg/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide anti-CRP antibody-modified affinity particles at a concentration of 0.3 wt%.
The bonding of the antibody to the particles was recognized by measuring the amount of a reduction in antibody concentration in the buffer solution having added thereto the antibody by BCA assay.
A change in fluorescence polarization was observed before and after the obtained anti-CRP antibody-modified affinity particles were mixed with a CRP antigen diluted with a MES buffer solution. An investigation was performed with the anti-CRP antibody-modified affinity particles fixed at 0.0001 mg/mL, and with CRP at an antigen concentration of from 0 pg/mL to 1,000 pg/mL. The observation was performed at normal temperature. A measurement method for fluorescence polarization is described later.

(Comparative Example 1)

The luminescent particles 7 were dispersed in a phosphate buffer solution to prepare a dispersion having a concentration of 0.01 mg/mL. 0.1 wt% of BSA was added to the dispersion, and the mixture was subjected to a reaction at 25°C overnight. After the reaction, centrifugation was performed, the supernatant was washed, and the precipitate was redispersed to provide a 0.001 mg/mL particle dispersion.

(Comparative Example 2)

The luminescent particles 8 were dispersed in a phosphate buffer solution to prepare a dispersion having a concentration of 0.01 mg/mL. 0.1 wt% of BSA was added to the dispersion, and the mixture was subjected to a reaction at 25°C overnight. After the reaction, centrifugation was performed, the supernatant was washed, and the precipitate was redispersed to provide a 0.001 mg/mL particle dispersion.

(Comparative Example 3)

Luminescent particles having a particle size of 400 nm and containing a europium complex (manufactured by Tamagawa Seiki Co., Ltd.) were diluted with pure water to 0.001 mg/mL to provide a particle dispersion.

(Evaluations of Products)

The products in Examples and Comparative Example were each subjected to such evaluations as described below.
The shape of the product was evaluated with an electron microscope (S5500 manufactured by Hitachi High-Technologies Corporation).
The average particle diameter of the product was evaluated by using dynamic light scattering (Zetasizer Nano S manufactured by Malvern).
The concentration of a suspension having the product dispersed therein was evaluated with a mass spectrometer (Thermo plus TG8120 manufactured by Rigaku Corporation).

A fluorescence spectrum of the product was measured with excitation light at 340 nm and with a polarizer placed in an optical path on each of an excitation side and a luminescence side. The measurement was performed with the direction of the polarizer on the excitation side being fixed, and the polarizer on the luminescence side being placed in a direction parallel or perpendicular to that on the excitation side. An apparatus used was a fluorescence spectrophotometer F-4500 manufactured by Hitachi High-Tech Science Corporation. The peak wavelength of observation light for analyzing polarized luminescence was set to 611 nm. The resultant data on polarized luminescence was analyzed with the equation (3) described above to determine the degree of polarization (polarization anisotropy) "r".

Nonspecific agglutination suppression evaluation of the product was performed as described below.

60 μl of a human serum solution diluted 15-fold with a buffer solution was added to 30 μl of each luminescent particle dispersion (3 mg/mL) produced in Examples 1 to 5 and Comparative Examples 1 to 3, and the mixture was kept at a temperature of 37°C for 5 minutes. An absorbance at 527 nm was measured before and after the temperature keeping, and the amount of change in absorbance before and after the temperature keeping was measured 3 times.

Table 2 shows the average value of the 3 times. Evaluation was performed as follows: when the amount of change in "absorbance×10,000" value was less than 1,000, it was determined that nonspecific agglutination was suppressed, and when the amount was 1,000 or more, it was determined that nonspecific agglutination occurred.

(Performance Evaluation)

According to the results of the nonspecific aggregation suppression evaluation, in all of Examples 1 to 5 and Comparative Examples 1 to 3, the change in absorbance was equal to or less than the specific numerical value, i.e., 0.01 or less, and thus it was recognized that the luminescent particles were capable of suppressing nonspecific adsorption.

The structures and physical properties of the particle materials of Examples 1 to 5 and Comparative Examples 1 to 3 are shown in Table 2.

[Table 2]

Table 2

| | Example 1 | Example 2 | Example 3 | Example 4 | Example 5 | Comparative Example 1 | Comparative Example 2 | Comparative Example 3 |
|---|---|---|---|---|---|---|---|---|
| Particle diameter of particles /nm | 25 | 105 | 204 | 269 | 349 | 140 | 206 | 400 |
| Rare earth complex | $(Phen)EuTTA_3$ | | | | | | | Europium complex |
| Surface | PVP-K30 | | | | | BSA | BSA | Diol |
| PDI | 0.086 | 0.077 | 0.01 | 0.09 | 0.082 | 0.129 | 0.173 | 0.233 |
| Polarization anisotropy "r" | 0.076 | 0.094 | 0.118 | 0.133 | 0.152 | 0.121 | 0.138 | 0.050 |

14

EP 4 349 887 A1

The luminescent particles of Examples 1, 2, 3, 4, and 5 had particle sizes of 25 nm, 105 nm, 204 nm, 269 nm, and 349 nm, respectively, and were also recognized to be monodispersed particles having pdi values of 0.086, 0.077, 0.01, 0.09, and 0.082, respectively, i.e., less than 0.1. The relationship of the values of the polarization anisotropy "r" determined from the results of the polarized light measurement with the particle sizes is shown in FIG. 2.

It was recognized from FIG. 2 that the particle size and the polarization anisotropy had an extremely high correlation. Meanwhile, the luminescent particles of Comparative Examples 1 and 2 had sizes of 140 nm and 206 nm, but had pdi values of 0.129 and 0.173, thus being recognized to have broad particle size distributions. Further, it was recognized that, in each of Comparative Examples 1 and 2, the polarization anisotropy "r" was located above the approximate curve shown in FIG. 2, and deviated from the linear correlation between the polarization anisotropy and the particle size owing to the broad particle size distribution.

The luminescent particles of Comparative Example 3 had a particle size of 400 nm, and had a polydispersity index pdi of 0.23 as measured by the dynamic light scattering method. It was recognized that the polarization anisotropy "r" of the luminescent particles was 0.05, located below the approximate curve shown in FIG. 2. In consideration of the luminescence lifetime of europium (from 0.1 millisecond to 1 millisecond) and the rotational relaxation time of 400 nm particles (about 6.4 milliseconds), the sample of Comparative Example 3 has such a particle size as to express sufficient polarization anisotropy, and can be predicted to have high polarization anisotropy. However, the low value of the polarization anisotropy means that the particles have small polarization anisotropy of the europium complex.

Table 3 and FIG. 3 show the results of quantification of the antigen-antibody reaction using the anti-CRP antibody, which was evaluated in Example 6, through use of a fluorescence depolarization method.

[Table 3]

Table 3

| | Example 6 | | | | |
|---|---|---|---|---|---|
| Particle diameter of sensitized particles /nm | 207 | | | | |
| CRP antigen concentration pg/mL | 0 | 1.0 | 10 | 100 | 1000 |
| Change in polarization anisotropy/$\Delta$r | 0 | 0.001 | 0.006 | 0.008 | 0.011 |

It was recognized from Table 3 and FIG. 3 that the amount of change in polarization anisotropy before and after the antigen-antibody reaction changed in accordance with the concentration of the CRP antigen. The antigen concentration of CRP is extremely small and in a region in which detection cannot be performed by a general latex agglutination method (ng/mL order). In addition, for luminescent particles using BSA, the numerical value of the polarization anisotropy deviates from prediction owing to a broad particle size distribution, and hence, in the case of detecting a target substance at a low concentration, it is difficult to accurately measure a change ratio.

Thus, it was revealed that the luminescent particle materials according to Examples of the present invention were polarized luminescent materials having high particle accuracy.

Accordingly, the particles according to Examples of the present invention can be used to provide particles for a specimen test for a fluorescence depolarization method having extremely high detection sensitivity. In particular, the particles are excellent in reducing effect on the fluctuation of the polarization anisotropy to be detected, and hence are suited for the detection of a target substance at a low concentration.

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2021-095971 filed on June 8, 2021, the description of which is incorporated herein by reference in its entirety.

[Reference Signs List]

1 particle substrate
2 hydrophilic layer
3 rare earth complex

**Claims**

1. A particle comprising a rare earth complex,

   wherein the particle has a particle size distribution of 0.1 or less in terms of polydispersity index measured by dynamic light scattering, and
   wherein the particle has a hydrophilic polymer on a surface thereof.

2. The particle according to claim 1, further comprising polystyrene and a siloxane bond.

3. The particle according to claim 1 or 2, wherein the particle has a polarization anisotropy <r> of 0.1 or more, which is determined by the following equation (4):
   [Math. 1]

$$\langle r \rangle = \frac{I_{vv} - G \cdot I_{VH}}{I_{vv} + 2 \cdot G \cdot I_{VH}}$$

$$G = \frac{I_{HV}}{I_{HH}}$$

$$\cdots \text{(Equation 4)}$$

   in the equation (4),

   <r> represents the polarization anisotropy,
   Ivv represents a luminescence intensity of a luminescence component having a vibration direction parallel to that of a first polarized light beam at a time of excitation by the first polarized light beam,
   $I_{VH}$ represents a luminescence intensity of a luminescence component having a vibration direction orthogonal to that of the first polarized light beam at the time of excitation by the first polarized light beam,
   $I_{HV}$ represents a luminescence intensity of a luminescence component having a vibration direction orthogonal to that of a second polarized light beam having a vibration direction orthogonal to that of the first polarized light beam at a time of excitation by the second polarized light beam,
   $I_{HH}$ represents a luminescence intensity of a luminescence component having a vibration direction parallel to that of the second polarized light beam having a vibration direction orthogonal to that of the first polarized light beam at the time of excitation by the second polarized light beam, and
   G represents a correction value.

4. The particle according to claim 1 or 2, wherein the rare earth complex contains at least one rare earth element selected from the group consisting of europium; terbium; neodymium; erbium; yttrium; lanthanum; cerium; samarium; gadolinium; dysprosium; thulium; ytterbium; and scandium.

5. The particle according to any one of claims 1 to 4,

   wherein the particle has a functional group capable of bonding a ligand on the surface thereof, and
   wherein the functional group is at least one selected from the group consisting of: a carboxy group; an amino group; a thiol group; an epoxy group; a maleimide group; and a succinimidyl group.

6. The particle according to any one of claims 1 to 5, wherein the hydrophilic polymer is polyvinylpyrrolidone.

7. The particle according to any one of claims 1 to 6, wherein the particle has an average particle diameter of 50 nm or more and 400 nm or less.

8. The particle according to any one of claims 1 to 7,
   wherein the particle has a difference between absorption spectra of 0.1 or less at an optical path of 10 mm and a wavelength of 572 nm before and after 30 $\mu$L of a dispersion of 0.1 wt% of the particle is added to 60 $\mu$L of a buffer solution mixed with 16 $\mu$L of serum and the mixture is left to stand at 37°C for 5 minutes.

9. A method of producing the particle of claim 1 or 2 comprising at least:

a first step of mixing a radically polymerizable monomer including styrene, a radical polymerization initiator, and a copolymer containing a unit having a pyrrolidone ring and a unit having a functional group capable of bonding a ligand with an aqueous medium to prepare an emulsion; and
a second step of polymerizing the radically polymerizable monomer by stirring the emulsion.

10. A method of producing a test reagent comprising a step of dispersing the particle of claim 1 or 2 in a dispersion medium.

11. The method of producing a test reagent according to claim 10, wherein the particle in the test reagent has an average particle diameter of 50 nm or more and 400 nm or less.

12. An affinity particle comprising:

the particle of claim 1 or 2; and
a ligand bonded to a functional group capable of bonding the ligand.

13. The affinity particle according to claim 12, wherein the ligand is at least one selected from the group consisting of: an antibody; an antigen; a protein; and a nucleic acid.

14. A test reagent for in vitro diagnosis comprising:

the affinity particle of claim 12 or 13; and
a dispersion medium for dispersing the affinity particle.

15. The test reagent for in vitro diagnosis according claim 14, wherein the test reagent is used for detection of an antigen or an antibody in a specimen by an agglutination method.

16. A test kit for in vitro diagnosis comprising:

the test reagent for in vitro diagnosis of claim 14 or 15; and
a case enclosing the test reagent.

17. A method of detecting a target substance in a specimen, the method comprising a step of mixing the test reagent for in vitro diagnosis of claim 14 or 15 with the specimen.

18. A method of detecting a target substance in a specimen by a fluorescence depolarization method, the detection method comprising the steps of

mixing the test reagent for in vitro diagnosis of claim 14 or 15 with the specimen to provide a mixed liquid;
irradiating the mixed liquid with polarized light; and
detecting polarized light caused to be emitted by the light with which the mixed liquid has been irradiated.

FIG. 1

# FIG. 2

# FIG. 3

| **INTERNATIONAL SEARCH REPORT** | International application No. |
|---|---|
| | **PCT/JP2022/022708** |

**A. CLASSIFICATION OF SUBJECT MATTER**

*C08J 3/12*(2006.01)i; *C09K 11/06*(2006.01)i; *G01N 33/545*(2006.01)i; *G01N 21/64*(2006.01)i; *G01N 21/78*(2006.01)i
FI:   C08J3/12; C09K11/06; C09K11/06 660; G01N21/64 A; G01N21/78 C; G01N33/545 A

According to International Patent Classification (IPC) or to both national classification and IPC

**B. FIELDS SEARCHED**

Minimum documentation searched (classification system followed by classification symbols)

C08J3/00 - 3/28, C08J99/00, C09K11/00 - 11/89, G01N33/48 - 33/98, G01N21/62 - 21/74, G01N21/75 - 21/83

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2019/132045 A1 (SEKISUI CHEMICAL COMPANY, LIMITED) 04 July 2019 (2019-07-04) claims, paragraphs [0015], [0016], [0019], [0021], [0024], [0032], [0039]-[0043], [0049], [0051], examples | 1-18 |
| A | JP 2008-111114 A (NATIONAL INSTITUTE OF ADVANCED INDUSTRIAL & TECHNOLOGY) 15 May 2008 (2008-05-15) entire text | 1-18 |
| A | JP 2015-120853 A (NATIONAL INSTITUTE OF ADVANCED INDUSTRIAL & TECHNOLOGY) 02 July 2015 (2015-07-02) entire text | 1-18 |
| A | JP 2015-010151 A (CHEIL INDUSTRIES, INCORPORATED) 19 January 2015 (2015-01-19) entire text | 1-18 |
| A | JP 2002-121421 A (SEKISUI CHEMICAL COMPANY, LIMITED) 23 April 2002 (2002-04-23) entire text | 1-18 |

☑ Further documents are listed in the continuation of Box C.        ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 August 2022** | **23 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 349 887 A1**

## INTERNATIONAL SEARCH REPORT

International application No.

**PCT/JP2022/022708**

### C. DOCUMENTS CONSIDERED TO BE RELEVANT

| Category* | Citation of document, with indication, where appropriate, of the relevant passages | Relevant to claim No. |
|---|---|---|
| A | JP 2001-181403 A (JSR CORPORATION; UNIVERSITY CLARKSON) 03 July 2001 (2001-07-03)<br>entire text | 1-18 |
| A | WO 2017/038138 A1 (SEKISUI PLASTICS COMPANY, LIMITED) 09 March 2017 (2017-03-09)<br>entire text | 1-18 |
| A | JP 60-099150 A (MITSUBISHI RAYON KABUSHIKI KAISHA) 03 June 1985 (1985-06-03)<br>entire text | 1-18 |
| A | JP 63-261806 A (KANEBO LIMITED) 28 October 1988 (1988-10-28)<br>entire text | 1-18 |
| A | JP 2011-057960 A (NATIONAL INSTITUTE OF ADVANCED INDUSTRIAL SCIENCE & TECHNOLOGY) 24 March 2011 (2011-03-24)<br>entire text | 1-18 |
| A | JP 2004-161849 A (DAINIPPON INK & CHEM. INCORPORATED) 10 June 2004 (2004-06-10)<br>entire text | 1-18 |
| A | JP 2020-159938 A (TORAY IND., INCORPORATED) 01 October 2020 (2020-10-01)<br>entire text | 1-18 |
| A | JP 2015-532930 A (OCEAN'S KING LIGHTING SCIENCE & TECHNOLOGY COMPANY, LIMITED) 16 November 2015 (2015-11-16)<br>entire text | 1-18 |
| A | WO 2021/062094 A1 (THOMAS JEFFERSON UNIVERSITY) 01 April 2021 (2021-04-01)<br>whole document | 1-18 |
| A | CN 104031647 A (HUIZHOU UNIVERSITY) 10 September 2014 (2014-09-10)<br>whole document | 1-18 |

Form PCT/ISA/210 (second sheet) (January 2015)

22

**INTERNATIONAL SEARCH REPORT**
Information on patent family members

International application No.

**PCT/JP2022/022708**

| Patent document cited in search report | | | Publication date (day/month/year) | Patent family member(s) | | | Publication date (day/month/year) |
|---|---|---|---|---|---|---|---|
| WO | 2019/132045 | A1 | 04 July 2019 | US 2021/0061820 A1 claims, paragraphs [0037], [0038], [0050]-[0052], [0059], [0060], [0081]-[0084], [0096]-[0110], [0117], [0119], examples | | | |
| JP | 2008-111114 | A | 15 May 2008 | US 2010/0056361 A1 whole document EP 2072462 A1 whole document | | | |
| JP | 2015-120853 | A | 02 July 2015 | (Family: none) | | | |
| JP | 2015-010151 | A | 19 January 2015 | WO 2014/208912 A1 whole document KR 10-2015-0009411 A whole document TW 201504352 A whole document | | | |
| JP | 2002-121421 | A | 23 April 2002 | (Family: none) | | | |
| JP | 2001-181403 | A | 03 July 2001 | US 5318797 A whole document EP 462388 A2 whole document | | | |
| WO | 2017/038138 | A1 | 09 March 2017 | CN 107949581 A whole document KR 10-2018-0048934 A whole document | | | |
| JP | 60-099150 | A | 03 June 1985 | US 4563494 A whole document EP 108622 A2 whole document | | | |
| JP | 63-261806 | A | 28 October 1988 | (Family: none) | | | |
| JP | 2011-057960 | A | 24 March 2011 | US 2012/0142808 A1 whole document | | | |
| JP | 2004-161849 | A | 10 June 2004 | (Family: none) | | | |
| JP | 2020-159938 | A | 01 October 2020 | (Family: none) | | | |
| JP | 2015-532930 | A | 16 November 2015 | US 2015/0240151 A1 whole document WO 2014/040229 A1 whole document EP 2896674 A1 whole document | | | |
| WO | 2021/062094 | A1 | 01 April 2021 | (Family: none) | | | |
| CN | 104031647 | A | 10 September 2014 | (Family: none) | | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0352575 B
- JP 2893772 B
- JP 2021095971 A